# EUROPEAN PATENT APPLICATION

(11) **EP 4 302 731 A1**
(43) Date of publication of application: **10.01.2024**
(21) Application number: 22315138.2
(22) Date of filing: 07.07.2022
(51) Int. Cl.: A61F 5/00, A61B 17/04

(54) **DEVICE FOR DELIVERING A GASTROINTESTINAL IMPLANT**

(71) Applicant: Caranx Medical SAS, 75013 Paris (FR)
(72) Inventor: SEJOR, Eric, 06000 Nice (FR); SMITS, Jonas Victor Harmen, 3360 Bierbeek (BE); BIADILLAH, Youssef, 78600 Maisons-Laffitte (FR); GRAY, Yonatan, 75011 Paris (FR); NAZ, Christophe, 77300 Fontainebleau (FR)
(74) Representative: Hepp Wenger Ryffel AG

(57) **Abstract**

An all-inclusive device 10 configured to deliver and anchor an implant 50 within the gastrointestinal tract 60 of a patient. The device 10 comprising an instrument body 11 configured to be inserted within a patient through a natural body opening; an implant installation system for anchoring and installing an implant 50; a probe 40 for guidance within the gastrointestinal tract 60.

## Description

The present invention relates to a device and method for delivering an implant within a patient, preferably within a tubular anatomical structure, according to the independent claims.

Research has shown the bariatric surgery is amongst the most efficient treatments for obesity. Studies show that bariatric surgery allows for increased weight loss as well as improvement of obesity induced comorbid conditions.

Bariatric interventions are often categorized into three categories: restrictive procedures; malabsorptive procedures and combination procedures. Amongst these, malabsorptive procedures are considered to give the best overall outcome allowing to reduce the patient's ability to absorb calories.

Bariatric surgeries in general tend to limit the patient's ability to consume or absorb calories and nutrients. And although bariatric interventions are becoming less and less invasive many issues still need addressing.

For example, one of the most efficient procedures for weight loss is a biliopancreatic diversion intervention where a large portion of the small intestines are by-passed by surgically disconnecting the duodenum from the stomach and surgically connecting the end part of the small intestine to the pylorus. However, altering the anatomical structure of the gastrointestinal tract is very strenuous on patients and.

Laparoscopic and endoscopic interventions are widely known for being generally advantageous procedures, yet they still present significant technical challenges.

Laparoscopic interventions require multiple instruments inserted within a patient by traversing the abdominal wall. This leads to relatively invasive procedures with multiple surgical incision points thus requiring more recovery time for the patient.

On the other hand, with a purely endoscopic procedure no surgical incisions are required. However, multiple instruments are still necessary and are inserted within a patient though the mouth for example. Having to use multiple, individually inserted instruments leads to a more complicated procedure for the practitioner as well as being potentially more harmful for the patient.

Implants have been proposed to avoid altering the anatomical structure of the gastrointestinal structure; however, the muscular tissues within the digestive system tend to induce migration of the implants thus making anchoring of implants complicated.

In WO 2006 0168 94 A1 an endoluminal device is described for delivering a gastrointestinal sleeve. The device has a container assembly and a gastrointestinal implant with an anchor at one end and a sleeve at the other. It is further described that the implant is placed within the container assembly for delivery to the gastrointestinal tract. In general, the sleeve of the implant is delivered to the gastrointestinal tract before the anchor. In addition, the anchor of the implant is self-expanding and is placed and secured in the duodenum of a patient.

In WO 2017 1326 79 A1 a gastrointestinal implant delivery system is proposed for placing an implant within the gastrointestinal tract. The inventions provide a delivery catheter including a lumen; a container assembly at the distal portion of the delivery catheter, the container assembly including a proximal capsule and a distal cap removably attachable thereto; and a gastrointestinal implant wherein the implant includes a sleeve.

In WO 2021 0806 40 A1 a system and method for anchoring and restraining gastrointestinal prostheses is described. The anti-migration anchor delivery system includes a first catheter configured to facilitate the delivery of an anti-migration anchor through tissue, a second catheter having a tissue engagement element configured to capture the tissue. The tissue engagement element is advanceable from a distal end of the second catheter. The tissue engagement element is operable to capture tissue such that the anti-migration anchor can be delivered through the tissue while the tissue is captured.

It is an object of the present invention to overcome or mitigate drawbacks of the prior art and in particular at least one of the above-mentioned disadvantages.

### Summary of the invention

A device according to the invention is adapted for delivering an implant with respect to a target site, preferably within the gastrointestinal tract. Some features relate to enabling efficient, through natural orifices and reliable delivery and anchoring of the implant.

In a first aspect the invention provides a device for delivering an implant to a target site in the gastrointestinal tissue. The device may comprise an instrument body that may be configured for insertion into a patient through a natural body opening to access the target site. The device may further provide an implant installation system supported through the instrument body, for anchoring and installing an implant. Additionally a probe supported through the instrument body for guidance within the gastro-intestinal tract, may be provided.

The probe may be, for example, an imaging probe for visualisation within the gastro-intestinal tract. Additionally or alternatively the probe may be a tissue probe for gathering information relative to surrounding tissues.

Providing a single all-inclusive device may facilitate the delivery of an implant to the gastrointestinal tract. It may also reduce the invasiveness of the procedure as only the single all-inclusive device would be inserted within the patient. A less invasive and all-inclusive device may reduce the duration of the procedure and is thus less strenuous on the patient. Provision of an imaging probe as part of the system can provide convenient and direct visualisation without the complication and less-di- . rect visualization associated with a separate imaging probe. Provision of a tissue probe may allow the gathering of information concerning the anatomical environment in which the probe is placed.

The probe may be configured to provide tissue information and/or image-guidance of some or all of the procedure.

The imaging probe may be, for example, an endoscopic camera; an endoscopic lens assembly; an ultrasound imaging probe.

The tissue probe may comprise sensors configured to provide information concerning tissue at, or near, the probe. The sensors may be one or more of the following: resistive force sensors; capacitive sensors; bioimpedance sensors; optical distance sensors; optical absorption sensors; photoplethysmography sensors.

In some embodiments the device may be directly operated by a practitioner. Additionally or alternatively the device may be remote-operated.

Additionally or alternatively, the device may operate in an at least partly autonomous manner.

The invention may comprise a control unit for operating one or more components of the device. The control unit may allow for autonomous and/or semi-autonomous operation of the device.

Optionally the device may comprise a positioning device for positioning one or more components of the device. For example, the positioning device may be able to position an instrument body of the delivery device with regards to a patient.

The control unit may be configured to operate the positioning of the device with regards to the patient. Additionally or alternatively the control unit may operate one or more components of the device once positioned within a patient.

The implant installation system may comprise an anchor delivery system operable to actively attach an anchor assembly to the gastrointestinal tissue.

The implant installation system may further comprise an implant deployment device for deploying the implant within the gastrointestinal tract.

In a second aspect of the invention, a device may be configured for delivering an implant to a target site in a gastrointestinal tissue. The device may comprise an instrument body that may be configured for insertion into a patient through a natural body opening to access the target site. Additionally the device may provide an anchor delivery system supported through the instrument body, for actively attaching an anchor assembly to the gastrointestinal tissue for anchoring the implant. Further additionally the device may have an implant deployment device supported through the instrument body, for deploying the implant within the gastrointestinal tract.

In either aspect, where used, the anchor delivery system and the implant deployment device may help avoid the need for additional instruments. Inserting the all-inclusive device through a natural body opening may help avoid any surgical incisions. This may help reduce the patient recovery time as well as the risk of infection.

In either aspect, where used, an anchor delivery system for actively attaching the anchor assembly to the target tissue may allow for a secure fixing of the implant with regards to the target tissue. For example, the gastrointestinal tract is subject to considerable muscle contractions. Active attachment can reduce risk of accidental displacement or migration of the implant in use, which is an important advantage.

The anchor delivery system may comprise a driver mechanism. The driver mechanism may be operable to actively drive one or more anchor assembly elements into anchoring engagement with gastrointestinal tissue.

In some embodiments the driver mechanism may have an at least partly circular configuration for driving and placing one or more anchor assembly elements in a circular anchoring engagement with the gastrointestinal tissue.

Additionally or alternatively, in some embodiments the driver mechanism may be at least partly configured to perforate a gastrointestinal tissue, optionally by driving one or more of the anchor assembly elements.

The driver mechanism may, for example, comprise a hollow needle. Optionally the needle may be pre-loaded with an anchor assembly element.

A circular anchoring engagement of one or more anchoring assembly elements may help distribute loads amongst plural elements. This may also further resist the muscle contractions of the gastrointestinal tract.

The anchor assembly may comprise an anchor element configured to at least partly traverse a gastrointestinal tissue. Additionally or alternatively the anchor assembly may comprise a base component configured for securing the position of the implant.

In some embodiments the anchor assembly comprises a plurality of anchor elements. Additionally or alternatively the anchor assembly may have a plurality of base components.

The one or more anchor elements and/or the one or more base components may help to avoid migration or dislodging of the implant and thus may provide a more secure anchoring of the gastrointestinal implant. This may help to avoid the need for corrective procedures.

Additionally integrating the anchoring assembly within the instrumentation body may help avoid accidental injuring of a patient's gastrointestinal tract during insertion of the instrument body.

One or more anchor elements may be placed in a circumferential manner with respect to the gastrointestinal tissue.

Optionally the anchor elements are placed at least partly on a pitch circle diameter with regards to the gastrointestinal tissue. For example, a plurality of anchor elements placed around, or near, a sphincter of the gastrointestinal tract may form a pitch-circle wherein the sphincter is in a central position.

The anchor elements may be any suitable means for anchoring an implant, for example, the anchor elements may be sutures; knotless suture anchors or staples. Additionally or alternatively the anchor element may be a staple like element.

Additionally or alternatively, the anchor elements may operate in a similar manner to fine barbs. In other words, the anchor elements may traverse a gastrointestinal tissue in a radially compressed state and then radially expand after traversing the tissue and/or implant.

Placing the anchor elements in a circumferential manner may allow for an even distribution of forces on the anchoring elements during contraction of the gastrointestinal tissue. This may help avoid tearing or damage to the tissue during the contractions of the muscle.

It may further help provide a more secure anchoring of the implant with respect to the gastrointestinal tissue.

Similarly, one or more base components may also have a circular geometry, for example, annular or ring shaped.

In some embodiments, the anchor assembly comprises a plurality of base components. At least one of the base components may be configured to translate with regards to another. Optionally both base components may be configured to translate with respect to each other.

Additionally or alternatively one or more base components may be configured to distally translate with respect to the instrument body.

Additionally or alternatively, in some embodiments one or more base components may be detachable from the instrument body. Optionally the base component configured to translate distally with regards to the instrument body may be the detachable base component.

Translation of one or more base components may grasp or sandwich gastrointestinal tissue. Optionally an annular portion of gastrointestinal tissue may be grasped.

Additionally or alternatively, in some embodiments at least one of the base components may be at least partly translucent for allowing a visual confirmation of the grasping of tissue.

The grasping of a gastrointestinal tissue by translation of one or more base components may facilitate the procedure as no further instrument is needed for positioning and grasping the tissue. Furthermore, the grasping of a tissue by the base component may further help to avoid incorrect positioning of the anchor assembly with respect to gastrointestinal tissue.

This also allows for a device specifically adapted to grasping a sphincter of the gastrointestinal tract, for example, the pyloric sphincter.

An at least partly translucent base component may help confirm that the gastrointestinal tissue is correctly grasped, when visualized by, for example, the imaging probe supported through the instrument body, where used. Incorrect grasping of the tissue could otherwise result in dislodgement and migration of the implant, which may need a further intervention to remove or correct.

This may further contribute to providing an all-inclusive device that enhances procedural efficiency and procedural outcomes. It may also reduce the invasiveness and risks of complication.

One or more anchor elements may be configured to traverse one or more base components, optionally traversing one or more openings in the one or more base components.

The one or more anchor elements traversing the base component may allow for anchoring the base component to the gastrointestinal tissue. Securing the anchor may help avoid dislodging of the implant. Providing such anchor elements may help provide a more patient-specific anchoring thus reducing the risk of dislodging of the implant.

The deployment device may comprise a deployment feature configured to translate within the instrument body. Additionally or alternatively the deployment feature may comprise a fluid pressure applicator for generating a fluid pressure. Further additionally or alternatively, the deployment feature may have a securing tab for restraining a self-deploying implant. Howsoever implemented, inclusion of the deployment feature may help provide an all-inclusive device and may reduce the need for additional instruments or devices during the intervention.

In some embodiments the deployment feature may be a guide-wire; a push rod or the probe, where used.

In some embodiments, a fluid pressure applicator may induce eversion of an implant, or extension of an inflatable hollow pusher body, for example, by progressive eversion.

Where used, the probe may be configured to translate with respect to the instrument body, and optionally to deploy the implant by such translation. Using the probe to deploy the implant may allow for a smaller device as no additional space-occupying deployment feature would be necessary. It may also allow for a visually guided progressive deployment of the implant, further enhancing procedural outcomes, even in difficult anatomies, and facilitating visual confirmation of correct deployment.

The implant may be a sleeve implant. Optionally the implant may be a gastrointestinal sleeve.

The sleeve implant may be adapted to bypass at least a portion of the gastrointestinal tract. Additionally, the sleeve implant may attach to a base component of the device. For example, the sleeve implant may be a duodenum sleeve attached to a base component secured at the pyloric sphincter and adapted to reduce the absorption of nutrients in the duodenum. In some embodiments the sleeve implant may be configured to evert distally with respect to the instrument body.

In some embodiments the implant may be configured to self-deploy along the gastrointestinal tract.

An all-inclusive device, such as described, may allow for an easier and less invasive delivery of a gastrointestinal sleeve. Including all the necessary elements within a single device may reduce the procedure time, thus the procedure may be less strenuous on the patient and the practitioner.

The gastrointestinal tissue may be a sphincter of the gastrointestinal tract, for example, at the pylorus.

Including elements specifically designed for a sphincter of the gastrointestinal tract, for example, the driver mechanism and the anchor elements in a circumferential configuration, may allow for a more precise and more adapted delivery of implants such as a gastrointestinal sleeve and a filament. This may help avoid complications such as tearing of tissue or migration of the implant.

The implant may be a filament and operation of the filament may reduce the size of the opening of the sphincter. Additionally or alternatively, operation of the filament may close the opening of the sphincter. Optionally the filament may be operated by tension.

The all-inclusive device may allow the use of a filament implant. This may allow for an easier procedure for reducing the size of or closing the sphincter opening. The all-inclusive device may provide a less invasive means of performing such a procedure. The device may help avoid unnecessary stress on the patient's body by limiting the number of instruments inserted within the patient.

In any aspect of the invention, at least a part of the instrument body may be configured to be placed in a first portion of the gastrointestinal tract of a patient and a second part may be positioned in a second part of the gastrointestinal tract. For -example, part of the instrument body may be configured to be placed in the stomach of a patient and a second part of the instrument body may be positioned in the duodenum of a patient.

Optionally, in any aspect of the invention, a first and second part of the anchor delivery system are configured to be placed in a first and second portion of the gastrointestinal tract.

In any aspect of the invention, the device may further comprise the implant to be installed by the device and/or the anchor assembly for securing the implant.

The implant may be a sleeve implant adapted to bypass at least a portion of the gastrointestinal tract. The sleeve implant may be a gastrointestinal sleeve, e.g. duodenum sleeve, adapted to reduce the absorption of nutrients in the duodenum.

The sleeve implant may be configured to attach to a base component of the device. The sleeve implant may be attached to the base component prior to procedure or during the procedure.

In a third aspect, the invention may provide a device for delivering an implant with respect of a gastrointestinal tissue. The device may comprise an instrument body configured for insertion within a patient and having at least one compartment.

The compartment may be configured to host a selection of one or more of an anchor delivery system for actively attaching an anchor assembly and securing an implant with regards to the gastrointestinal tissue; a deployment feature for deploying an implant within the gastrointestinal tract; an implant to be deployed with the deployment feature; a probe, optionally an imaging probe for visualisation within the gastrointestinal tract.

Hosting one or more of an anchor delivery system; a deployment feature; an implant; a probe within a compartment of the device may help provide an all-inclusive device for delivering and anchoring an implant with respect of a gastrointestinal tissue. Accommodating one or more of the above-mentioned elements in a compartment of the instrument may help avoid needing to insert additional instruments for delivering or anchoring an implant with respect to the gastrointestinal tissue. It may further help avoid accidental injury to the patient during insertion of the device.

In a fourth aspect the invention may provide a method for delivering an implant within a gastrointestinal tract. The method may optionally use a device according any of the aspects.

The method may further comprise a step for operating an anchor delivery system for attaching an anchor assembly to a gastrointestinal tissue.

Additionally or alternatively the method may provide a step for engaging a deployment feature to deploy an implant Additionally or alternatively the method may comprise a step for delivering an implant to the gastrointestinal tract Further additionally or alternatively, the method may have a step for working a probe, optionally an imaging for internal visualisation and/or a tissue probe for gathering tissue information.

### Brief Description of the Drawings

- Fig. 1: is a schematic perspective view of a device for delivering an implant with respect to a target tissue.
- Fig. 2: is a schematic longitudinal cross-section of an alternative embodiment of a device for delivering an implant with respect to a target tissue.
- Fig. 3: is a schematic top-view of an anchored implant with respect of a target tissue.
- Fig. 4a-b: are schematic illustrations of a method of delivering an implant to a target tissue.
- Figs. 5a: is a side-view of a method of anchoring an implant with respect to the target tissue.
- Fig. 5b: is a top-view of fig. 5a.
- Figs. 5c: is a side-view of a method of anchoring an implant with respect to the target tissue.
- Fig. 5d: is a top-view of the anchoring method of fig. 5c.

### Detailed Description of Embodiments

Non-limiting embodiments of the invention are now described by way of example only.

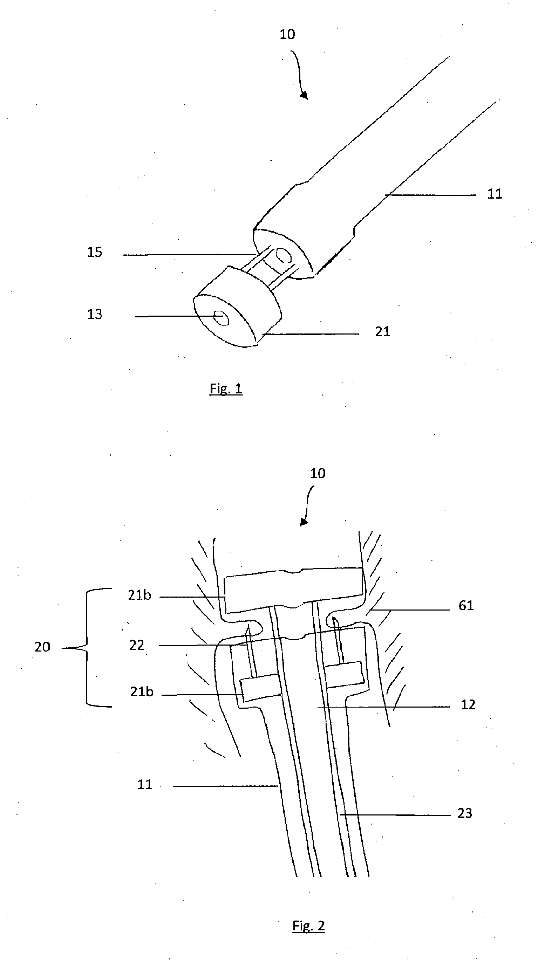

Referring to fig. 1, a device 10 according to the invention comprises an instrument body 11. The device 10 has an operator end for operating the device 10 and a working end for insertion within a patient. The instrument body 11 of the device 10 is configured to be inserted within a patient, preferably through a natural body opening.

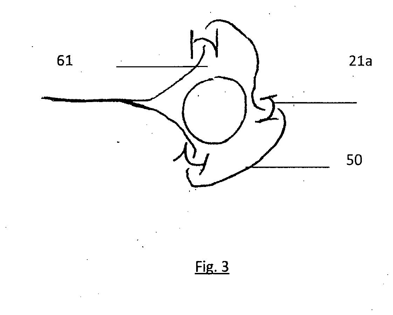

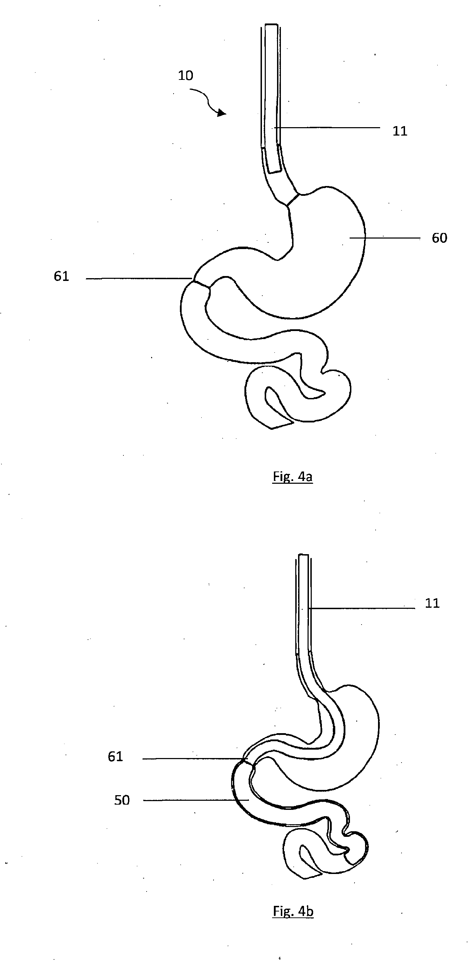

In this embodiment, the instrument body 11 has an opening 13 for delivering an implant 50 (see Fig. 3) to a target tissue. The target tissue is, for example, the gastrointestinal tract 60 of a patient (see Fig. 4a). The opening 13 is in communication with a compartment 12 (see fig. 2) of the instrument body 11.

This allows the passage of the implant 50 within the compartment 12 of the instrument body 11 to be delivered to the target tissue.

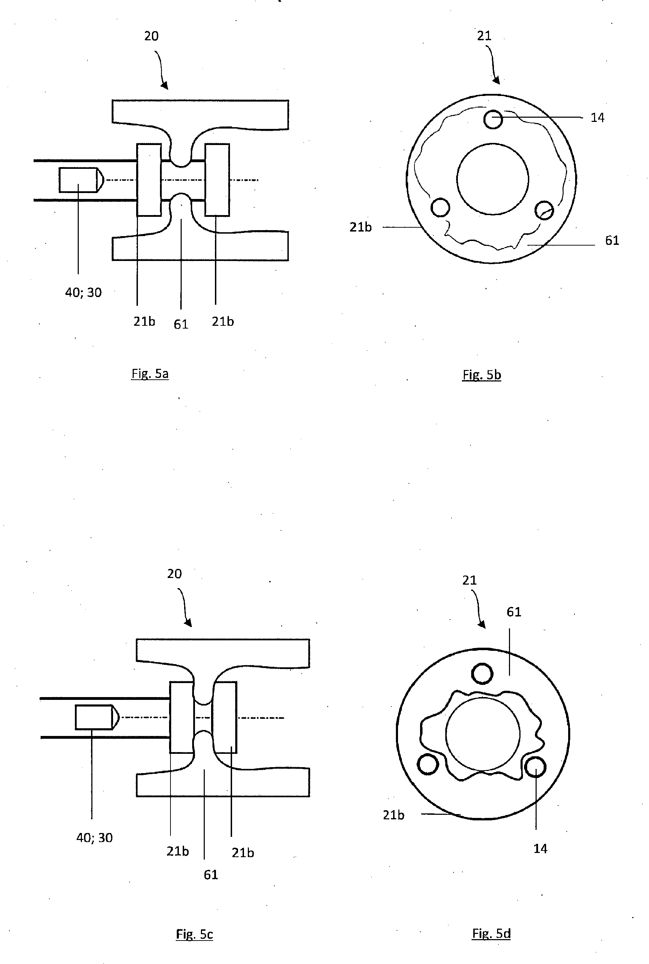

The device 10 provides an implant installation system (seen in figs. 2, 5a and 5c). The implant installation system provides one or more movers 23 for anchoring and installing an implant 50. The movers 23 are configured to induce the translation of one or more components of the implant installation system. The movers 23 are operated by means of the operator end of the device 10.

Illustrated in fig. 2, a base component 21b is connected distally to the instrument body 11 by at least one mover 23. The base component 21b is configured to translate distally upon operation of an operator end. The translation of the base component 21b is induced by the translation motion of at least one mover 23.

The instrument body 11 comprises a compartment 12 for housing one or more components of the device 10. The mover(s) 23 are housed within the compartment 12 of the instrument body 11. The mover(s) 23 are configured to extend within the instrument body 11.

The compartment(s) 12 is further configured to accommodate one or more components of the instrument body 11. For example, in an alternative embodiment, the compartment 12 houses a probe 40 (see fig. 5a and 5c). In a further alternative embodiment not shown, the compartment 12 also houses an implant 50 to be delivered within the target tissue.

The implant installation system comprises an anchor delivery system 20 comprising one or more driver mechanisms 22 and one or more anchor assembly elements. The driver mechanism 22 is configured to actively engage at least one anchor assembly element with the target tissue, for example, the gastrointestinal tissue 61. The driver 22 actively drives the anchor assembly 21 (see fig. 5b and 5d) into anchoring engagement with the target tissue.

The anchor assembly 21 as shown is configured to connect an implant 50 to the gastrointestinal tissue 61. The anchor assembly 21 comprises a plurality of base components 21b and a plurality of anchor elements 21a (see Fig. 3).

The base component 21b is configured to grasp a portion of the gastrointestinal tissue 61 to secure the position of the implant 50 and translates distally to this effect. The anchor element 21a is configured to at least partly traverse a gastrointestinal tissue 61 and at least one base component 21b.

The driver mechanism 22 is a stapler, or stapler-like, feature and has an at least partly circular configuration for driving a plurality of anchor elements 21a for engaging with a gastrointestinal tissue 61. The at least partly circular configuration of the driver engages the anchor elements 21a, e.g. staples, in a circular configuration.

Referring to fig. 3 the anchor elements 21a are disposed in a partly circular manner with respect to a gastrointestinal tissue, for example, a sphincter.

The implant 50 is a filament implant configured to close or reduce the size of the sphincter opening upon operation of the filament. The sphincter is closed or the opening reduced by inducing tension on the filament.

The anchor elements 21a are staple-like and configured to traverse the gastrointestinal tissue 61 and anchor the filament implant with respect to the sphincter.

Depicted in fig. 4a-b the device 10 is introduced (fig. 4a) within the gastrointestinal tract 60 of a patient using a natural body opening, for example, the mouth. The instrument body 11 is navigated to the target gastrointestinal tissue, for example, the pyloric sphincter 61 where the implant 50 (fig. 4b) will be anchored and deployed using the device 10. In this example, the implant 50 (fig. 4b) comprises a gastrointestinal bypass sleeve for anchoring at the pyloric sphincter 61, and for extending into the small intestine.

Illustrated in fig. 5a-d is an exemplary embodiment of the anchor delivery system 20 of the instrument body 11. The instrument body 11 is inserted within a patient and positioned at the target location, for example, a sphincter such as the pyloric sphincter 61. The instrument body 11 is further positioned so that a first base component 21b of the anchor delivery system 20 is positioned within a first portion of the gastrointestinal tract 60, e.g. the stomach, and a second base component 21b of the anchor assemble system positioned in a second portion of the gastrointestinal tract 60, e.g. the duodenum.

The anchor assembly 21 has a first configuration wherein the base components 21b in the form of plates are positioned in a non-engaging manner with respect to the sphincter (see fig. 5a and 5b) and a second configuration wherein the base components 21 grasp an annular portion of the gastrointestinal tract 60 (see fig. 5c and 5d), for example, an annular portion of tissue around the sphincter 61. One, or both, base components 21b are configured to translate to grasp the gastrointestinal tissue, e.g. the sphincter 61.

In figs. 5b and 5c the base components 21b comprise a plurality of openings 14 (fig. 5b and 5d) to be traversed with a plurality of anchoring elements. The plurality of openings 14 of the plurality of base components 21b are configured to axially align. Additionally, at least one opening is configured to allow the delivery of an implant 50 to the gastrointestinal tract 60 (see fig. 4b) by a deployment device 30. In this depicted embodiment, the imaging probe 40 is used as the deployment device 30.

Furthermore, one, or more, of the base components 21b can be translucent for allowing imaging probe 40 visualisation of the grasped gastrointestinal tissue 61.

The imaging probe 40 is configured to translate axially, for visualisation of and along the gastrointestinal tissue 61. In fig. 5b the imaging probe 40 shows the base component 21b positioned with respect to the sphincter in a non-engaged configuration. In fig. 5d the imaging probe 40 shows a tissue correctly grasped by the base components 21b in a grasping configuration. The probe 40 can also be used as a pusher or deployer for deploying an implant 50 in the form of a bypass sleeve (e.g. Figs. 4b, 5a and 5c). Such operation can avoid the need for a separate deployer or pusher in the instrument, and can also enable direct visualisation during deployment if desired.

It will be appreciated that the foregoing description is merely illustrative of preferred forms of the invention, and that many modifications and equivalents may be used that would all fall within the inventive scope that has been disclosed.

## Claims

1. Device (10) for delivering an implant (50) to a target site in a gastrointestinal tract, the device (10) comprising:
(i) an instrument body (11) configured for insertion into a patient through a natural body opening to access the target site;
(ii) an implant installation system supported through the instrument body (11), for anchoring and installing an implant (50); and
(iii) a probe (40) supported through the instrument body (11) for guidance within the gastrointestinal tract (60).

2. Device (10) according to claim 1, wherein the implant installation system comprises an anchor delivery system (20) operable to actively attach an anchor assembly (21) to the gastrointestinal tissue (61).

3. Device (10) according to claim 1 or 2, wherein the implant installation system comprises an implant deployment device (30) for deploying the implant (50) within the gastrointestinal tract (60).

4. Device (10) for delivering an implant (50) to a target site in gastrointestinal tissue, the device (10) comprising:
(i) an instrument body (11) configured for insertion into a patient through a natural body opening to access the target site;
(ii) an anchor delivery system (20) supported through the instrument body (11), for actively attaching an anchor assembly (21) to the gastrointestinal tissue (61) for anchoring the implant (50); and
(iii) an implant deployment device (30) supported through the instrument body (11), for deploying the implant (50) within the gastrointestinal tract.

5. Device (10) according to claim 2, 3, or 4, wherein the anchor delivery system (20) comprises a driver mechanism (22) operable to actively drive one or more anchor assembly (21) elements into anchoring engagement with gastrointestinal tissue (61).

6. Device (10) according to claim 2, 3, 4 or 5, wherein the anchor assembly (21) connects an implant (50) to a gastrointestinal tissue (61) and comprises a selection of one or more of: an anchor element (21a) configured to at least partly traverse a gastrointestinal tissue (61); and a base component (21b) configured for securing the position of the implant.

7. Device (10) according to claim 6, wherein one or more anchor elements (21a) are placed in a circumferential manner with respect to the gastrointestinal tissue.

8. Device (10) according to claim 5 or 6, wherein the anchor assembly (21) comprises a plurality of base components (21b) and further wherein at least one of the base components (21b) is configured to translate with regard to another, optionally both base components (21b) being configured to translate with respect to each other

9. Device (10) according to claim 8, wherein translation of one or more base components (21b) grasps a gastrointestinal tissue (61), optionally an annular portion of gastrointestinal tissue

10. Device (10) according to any of claims 4 to 9, wherein one or more anchor elements (21a) are configured to traverse one or more base components, optionally traversing one or more openings (13) in the one or more base components (21b) .

11. Device (10) according to claim 3 or 4 or any claim dependent thereon, wherein the deployment device comprises one or more of:
(i) a deployment feature configured to translate within the instrument body;
(ii) a fluid pressure applicator for generating a fluid pressure;
(iii) a securing tab for restraining a self-deploying implant.

12. Device (10) according to claim 1 or any claim dependent thereon, wherein the probe (40) is configured to translate with respect to the instrument body (11), optionally wherein translation of the probe (40) deploys the implant (50) .

13. Device (10) according to any preceding claim, wherein the implant (50) is a sleeve implant, optionally a gastrointestinal sleeve attachable to at least one base component (21b) .

14. Device (10) according to any preceding claim, wherein the gastrointestinal tissue (61) is a sphincter of the gastrointestinal tract.

15. Device (10) according to any claims 1 to 4 and 10 to 14 wherein an implant (50) is a filament and operation, optionally by tension, of the filament reduces the size and/or closes the opening of the sphincter.

16. A device (10) according to any preceding claim, further comprising at least one, optionally both, of:
(i) the implant (50) to be installed by the device (10), and
(ii) the anchor assembly (21) for securing the implant.

17. Method for delivering an implant (50) within a gastrointestinal tract, optionally using a device (10) according to any claims 1 to 16, the method comprising inserting an instrument body (11) through a natural body opening to access a target site within the gastrointestinal tract, and the method further comprising one or more of:
(i) operating an anchor delivery system (20) supported through the instrument body (11), to actively attach an anchor assembly (21) to gastrointestinal tissue (61); and/or
(ii) operating a probe (40) supported through the instrument body (11).
